# FASCICULE DE BREVET EUROPEEN

(11) **EP 1 390 509 B1**
(45) Date de publication et mention de la délivrance du brevet: **20.06.2007**
(21) Numéro de dépôt: 02740803.8
(22) Date de dépôt: 21.05.2002
(51) Int. Cl.: C12N 15/54, C12Q 1/68

(54) **SEQUENCE DU GENE RPOB DE LA BACTERIE TROPHERYMA WHIPPELII ET OLIGONUCLEOTIDE POUR LE DIAGNOSTIC MOLÉCULAIRE DE LA MALADIE DE WHIPPLE**
SEQUENZ DES RPOB GENS DER TROPHYREMA WHIPELII BAKTERIE UND OLIGONUKLEOTID FÜR DIE MOLEKULARE DIAGNOSTIK DER KRANKHEIT VON WHIPPLE
SEQUENCE OF THE TROPHERYMA WHIPPELII BACTERIA RPOB GENE AND OLIGONUCLEOTIDE FOR MOLECULAR DIAGNOSIS OF WHIPPLE'S DISEASE

(30) Priorité: 21.05.2001 FR 0106641
(43) Date de publication de la demande: 25.02.2004
(73) Titulaire: Université de la Méditerranée, Aix-Marseille II, 13284 Marseille Cedex 07 (FR)
(72) Inventeur: RAOULT, Didier, F-13008 Marseille (FR); DRANCOURT, Michel, F-13012 Marseille (FR)
(74) Mandataire: Domange, Maxime
(86) Numéro de dépôt international: PCT/FR2002/001698
(87) Numéro de publication internationale: WO 2002/095033

(56) Documents cités:
- WO-A-00/58440
- US-A- 6 074 865
- HINRIKSON H P ET AL: "DETECTION OF THREE DIFFERENT TYPES OF 'TROPHERYMA WHIPPELII' DIRECTLY FROM CLINICAL SPECIMENS BY SEQUENCING, SINGLE-STRAND CONFORMATION POLYMORPHISM (SSCP) ANALYSIS AND TYPE-SPECIFIC PCR OF THEIR 16S-23S RIBOSOMAL INTERGENIC SPACER REGION" INTERNATIONAL JOURNAL OF SYSTEMATIC BACTERIOLOGY, SOCIETY FOR GENERAL MICROBIOLOGY, READING, GB, vol. 49, 1999, pages 1701-1706-2006, XP000914233 ISSN: 0020-7713
- DIDIER RAOULT ET AL.: "Cultivation of the Bacillus of Whipple's disease" THE NEW ENGLAND JOURNAL OF MEDICINE, vol. 342, no. 9, 2 mars 2000 (2000-03-02), pages 620-625, XP000913986
- HANS-PETER KLENK ET AL.: "DNA-dependent RNA polymerase subunit B as a tool for phylogenetic reconstructions: Branching topology of the archaeal domain" JOURNAL OF MOLECULAR EVOLUTION, vol. 38, 1994, pages 420-432, XP001064827
- MICHEL DRANCOURT ET AL.: "rpoB sequence analysis of cultured Tropheryma whippelii" JOURNAL OF CLINICAL MICROBIOLOGY, vol. 39, no. 7, juillet 2001 (2001-07), pages 2425-2430, XP002225067 & DATABASE EMBL [en ligne] Entry AF243072, 24 septembre 2001 (2001-09-24) DRANCOURT M. ET AL.: "Bacterium DR-2001 RpoB gene"

### LISTE DE SEQUENCES

<110> Université de la Méditerranée (Aix-Marseille II)
<120> Séquence du gène rpoB de la bactérie Tropheryma whippelli et oligonucléotide pour le diagnostic moléculaire de la maladie de Whipple
<130> H52439-4
<140>
   <141>
<160> 24
<170> PatentIn Ver. 2.1
<210> 1
   <211> 19
   <212> ADN
   <213> Séquence artificielle
<220>
   <223> Description de la séquence artificielle:oligonucléotide
<400> 1
   tnatgggnnc naanatgca 19
<210> 2
   <211> 20
   <212> ADN
   <213> Séquence artificielle
<220>
   <223> Description de la séquence artificielle:oligonucléotide
<400> 2
   gcccancatt ccatntcncc 20
<210> 3
   <211> 610
   <212> ADN
   <213> Tropheryma whippelii
<400> 3
   gagtcccctg gtcggcacag gtatggagcg gtatgtagcg atcgatgcgg gtgatgtttt 60
   aattgccgag gatccgggca ttgtggggga tgtttccgct gatgttgtca ctgtcaagca 120
   ggatgacggg aaacatcgcg actaccatgt tggtaaattt gttcgttcaa atcagggcaa 180
   ctgttacaac cagcnagttg tggtccgatc cggagatcgt gtataaaaag gtacagttct 240
   tgcacatggt ccatgtactg acaaaggtga gcttagtctt ggtagaaatc ttctggttgc 300
   tttcatgccc tgggagggct ataactttga ggatgcgata attatcagcc agaatttggt 360
   caaggacgac accctttcnt caatccacat agaagaacat gaggttagca cccgggatac 420
   gaagctgggc agtgaagaaa taacgcgaga ccttccgaat gtaagcatgg attacataaa 480
   ggacttggac gaacggggta ttatccggat tggcgctgag gttggccctg gggacatttt 540
   ggttggtaag gtgaccccaa agggcgagac cgaactcagc gcggaagagc gtttgctgag 600
   ggctatcttt                                                        610
<210> 4
   <211> 2750
   <212> ADN
   <213> Tropheryma whippelii
<400> 4
   atcccccata gaagattcgg ctggcaatat gcagcttaat ttctccaagc cggttctgga 60
   ggccgaagag ttgagcgtaa gggaatgccg tgttcgaggc aggacctatt cggcgcccct 120
   gtatgttgag gccgagttca tgaatcatga tacgggcgag ataagactca gacggttttt 180
   atgggcgatt tcccgcttat gactgacaag ggtacattcg tcataaacgg caccgagaga 240
   gttgttgtct ctcagcttgt tcgcagccct ggtgtttatt ttgagcgcac gccggaaaaa 300
   aacagtgaaa aggatctttt ttcgggcaga ataatccccg ctcgcggtgc ttggctagaa 360
   ttcgaagttg acaggcatga ccagcttggc gttagggttg acaggaagcg caggcagccg 420
   gttattttct ttctgagagc aattggcatg actgatgatg agatcaggga tgcatttggc 480
   gagtttgaat caataagcgt ccagcacgaa aagaatattg ggctgtccag agatgacgcg 540
   ctccgggaaa tataccgtcg cgttcgtccg ggggagcagg catcggctga ggctgggcgt 600
   gcactcttag agaattttta ctttaccagc agacgttttg acctggcaag ggttggaagg 660
   tacaaagtaa atcgcaaact cggtgttgat gttgatccaa ctcggatggt tcttacgagg 720
   tcggatatta tcgcaacaat tcgttatctc gcggccttgc atctcggttt ctccgaggtt 780
   gcggtgctaa acagcaacaa gagtgtacca atttcaaccg atgatattga ccatcttggt 840
   aataggcgca ttagaccggt tggtgagttg gtgcaaaacc agctccgggc tggtctggcc 900
   agaatggagc gggttgtgag agagcgcatg acaacccagg atatagaggc gattatcccg 960
   cagacactca taaatgtcat gccaattgtt gcagcgctga aggagtttta tggcaccagc 1020
   cagctctcgc agtttatgga tcagaataac ccccttgccg gccttacgca caagaggcgc 1080
   ttgtcagctc ttggccccgg tggactgtcc cgcgaacgtg ccggtgttga ggttcgagac 1140
   gtaaatccca gtcattacgg caggatgtgt ccgatcgaaa ccccagaagg tccaaatatt 1200
   ggccttatag ggtctttagc gtgctattcc agggtgaaca gtttcggctt tatagaaacc 1260
   ccatatagac gtgttgtcaa cggaaaagtc acagacgata ttgagtatat gacagcaaca 1320
   caggaagatg agcatgcgat tgcccaggca agcactccgc tgcgaccgga caactcgttt 1380
   gttgatgagc gtgttctggt tcgccgaaaa ggtggagagg tcgaggttgt accggccgat 1440
   caggttgatt atatggatgt gtctggacgt cagatggtgt ctgttgcaac ttcgcttata 1500
   cccttccttg aacataatga cgctaaccgt gctctcatgg gatcgaatat gcagagacag 1560
   gctgtaccac ttctggttac ggagagtccc ctggtcggca caggtatgga gcggtatgta 1620
   gcgatcgatg cgggtgatgt tttaattgcc gaggatccgg gcattgtggg ggatgtttcc 1680
   gctgatgttg tcactgtcaa gcaggatgac gggaaacatc gcgactacca tgttggtaaa 1740
   tttgttcgtt caaatcaggg caactgttac aaccagcnag ttgtggtccg atccggagat 1800
   cgtgtagaaa aaggtacagt tcttgcacat ggtccatgta ctgacaaagg tgagcttagt 1860
   cttggtagaa atcttctggt tgctttcatg ccctgggagg gctataactt tgaggatgcg 1920
   ataattatca gccagaattt ggtcaaggac gacacccttt cgtcaatcca catagaagaa 1980
   catgaggtta gcacccggga tacgaagctg ggcagtgaag aaataacgcg agaccttccg 2040
   aatgtaagca tggattacat aaaggacttg gacgaacggg gtattatccg gattggcgct 2100
   gaggttggcc ctggggacat tttggttggt aaggtgaccc caaagggcga gaccgaactc 2160
   agcgcggaag agcgtttgct gagggctatc tttaacgaga agagcatgga ggttcgcgac 2220
   acaagtttga aggtcccaca cgggcagcag gggaccgtaa tcgatgttaa gttgtttgat 2280
   gcggttgacg gtgaagataa gctgggtgcc ggcataaatc agcgggttgt tgtgtacata 2340
   gcgcataagc gcaagattac ggagggagat aagcttgctg ggcgccatgg caacaagggt 2400
   gttatttcaa agatccttcc ggtagaggat atgcccttca tggctgatgg gacccccgtt 2460
   gatataatcc ttaatccgct cggcgtgccc gcgcgtatga acttcgggca ggttttggaa 2520
   acccatttgg ggtggatctc taagcaagga tggaaaatag agggtgatcc tgattgggca 2580
   aaagatattc gggtgcgcga ggcgcagcct gatagtaggg tttcaagtcc ggtttttgat 2640
   ggaatttccg aaggggagat aaccgggctt ttttcttctg tatttccaaa ccgtgatggt 2700
   gagcgtgctg tcggttctga tggtaaggct attctctatg atggccgcac            2750
<210> 5
   <211> 20
   <212> ADN
   <213> Séquence artificielle
<220>
   <223> Description de la séquence artificielle:amorce
<400> 5
   cttatcacgc ccgtatcatg 20
<210> 6
   <211> 25
   <212> ADN
   <213> Séquence artificielle
<220>
   <223> Description de la séquence artificielle:amorce
<400> 6
   ccagtcaaaa ctatcgagct gcaaa 25
<210> 7
   <211> 19
   <212> ADN
   <213> Séquence artificielle
<220>
   <223> Description de la séquence artificielle:amorce
<400> 7
   ttctctatga tggccgcac 19
<210> 8
   <211> 22
   <212> ADN
   <213> Séquence artificielle
<220>
   <223> Description de la séquence artificielle:amorce
<400> 8
   gcagcgcttc ggagagatgg ag 22
<210> 9
   <211> 3761
   <212> ADN
   <213> Tropheryma whippelii
<400> 9
   atgccaatgt ggcgttataa ctgccccagt gttattcctg taaacgcgcg gggtgctgta 60
   aagtgtcgca gcttgctatg ctgccctgga tttgctacaa tcgttctcgc tgtctcgcca 120
   cttgccttca tattgcggtc ggctctggct ttgtcggggt tttttccctc ttgcccgctt 180
   tgggtgtctt tgtttggggt aggttttttg gcttctgcta agggtaaaag ggtttgtgcg 240
   ataggtcgct cctctctcgg taagatctct gatccgcttg aggttccgaa ccttcttgat 300
   ttgcagctcg atagttttga ctggctcata ggcggcccta ggtggcgcgt cgcccttgat 360
   gcttaccgca aaaatccgtc aggcgcccca attgccgaaa agagtgggct ggatgaggtg 420
   tttgatgaga tatcccccat agaagattcg gctggcaata tgcagcttaa tttctccaag 480
   ccggttctgg aggccgaaga gttgagcgta agggaatgcc gtgttcgagg caggacctat 540
   tcggcgcccc tgtatgttga ggccgagttc atgaatcatg atacgggcga gataagactc 600
   agacggtttt tatgggcgat ttcccgctta tgactgacaa gggtacattc gtcataaacg 660
   gcaccgagag agttgttgtc tctcagcttg ttcgcagccc tggtgtttat tttgagcgca 720
   cgccggaaaa aaacagtgaa aaggatcttt tttcgggcag aataatcccc gctcgcggtg 780
   cttggctaga attcgaagtt gacaggcatg accagcttgg cgttagggtt gacaggaagc 840
   gcaggcagcc ggttattttc tttctgagag caattggcat gactgatgat gagatcaggg 900
   atgcatttgg cgagtttgaa tcaataagcg tccagcacga aaagaatatt gggctgtcca 960
   gagatgacgc gctccgggaa atataccgtc gcgttcgtcc gggggagcag gcatcggctg 1020
   aggctgggcg tgcactctta gagaattttt actttaccag cagacgtttt gacctggcaa 1080
   gggttggaag gtacaaagta aatcgcaaac tcggtgttga tgttgatcca actcggatgg 1140
   ttcttacgag gtcggatatt atcgcaacaa ttcgttatct cgcggccttg catctcggtt 1200
   tctccgaggt tgcggtgcta aacagcaaca agagtgtacc aatttcaacc gatgatattg 1260
   accatcttgg taataggcgc attagaccgg ttggtgagtt ggtgcaaaac cagctccggg 1320
   ctggtctggc cagaatggag cgggttgtga gagagcgcat gacaacccag gatatagagg 1380
   cgattatccc gcagacactc ataaatgtca tgccaattgt tgcagcgctg aaggagtttt 1440
   atggcaccag ccagctctcg cagtttatgg atcagaataa cccccttgcc ggccttacgc 1500
   acaagaggcg cttgtcagct cttggccccg gtggactgtc ccgcgaacgt gccggtgttg 1560
   aggttcgaga cgtaaatccc agtcattacg gcaggatgtg tccgatcgaa accccagaag 1620
   gtccaaatat tggccttata gggtctttag cgtgctattc cagggtgaac agtttcggct 1680
   ttatagaaac cccatataga cgtgttgtca acggaaaagt cacagacgat attgagtata 1740
   tgacagcaac acaggaagat gagcatgcga ttgcccaggc aagcactccg ctgcgaccgg 1800
   acaactcgtt tgttgatgag cgtgttctgg ttcgccgaaa aggtggagag gtcgaggttg 1860
   taccggccga tcaggttgat tatatggatg tgtctggacg tcagatggtg tctgttgcaa 1920
   cttcgcttat acccttcctt gaacataatg acgctaaccg tgctctcatg ggatcgaata 1980
   tgcagagaca ggctgtacca cttctggtta cggagagtcc cctggtcggc acaggtatgg 2040
   agcggtatgt agcgatcgat gcgggtgatg ttttaattgc cgaggatccg ggcattgtgg 2100
   gggatgtttc cgctgatgtt gtcactgtca agcaggatga cgggaaacat cgcgactacc 2160
   atgttggtaa atttgttcgt tcaaatcagg gcaactgtta caaccagcna gttgtggtcc 2220
   gatccggaga tcgtgtagaa aaaggtacag ttcttgcaca tggtccatgt actgacaaag 2280
   gtgagcttag tcttggtaga aatcttctgg ttgctttcat gccctgggag ggctataact 2340
   ttgaggatgc gataattatc agccagaatt tggtcaagga cgacaccctt tcgtcaatcc 2400
   acatagaaga acatgaggtt agcacccggg atacgaagct gggcagtgaa gaaataacgc 2460
   gagaccttcc gaatgtaagc atggattaca taaaggactt ggacgaacgg ggtattatcc 2520
   ggattggcgc tgaggttggc cctggggaca ttttggttgg taaggtgacc ccaaagggcg 2580
   agaccgaact cagcgcggaa gagcgtttgc tgagggctat ctttaacgag aagagcatgg 2640
   aggttcgcga cacaagtttg aaggtcccac acgggcagca ggggaccgta atcgatgtta 2700
   agttgtttga tgcggttgac ggtgaagata agctgggtgc cggcataaat cagcgggttg 2760
   ttgtgtacat agcgcataag cgcaagatta cggagggaga taagcttgct gggcgccatg 2820
   gcaacaaggg tgttatttca aagatccttc cggtagagga tatgcccttc atggctgatg 2880
   ggacccccgt tgatataatc cttaatccgc tcggcgtgcc cgcgcgtatg aacttcgggc 2940
   aggttttgga aacccatttg ggtggatctc taagcaaggg atggaaaata gagggtgatc 3000
   ctgattgggc aaaagatatt cgggtgcgcg aggcgcagcc tgatagtagg gtttcaagtc 3060
   cggtttttga tggaatttcc gaaggggaga taaccgggct tttttcttct gtatttccaa 3120
   accgtgatgg tgagcgtgct gtcggttctg atggtaaggc tattctctat gatggccgca 3180
   cgggggagcc cttcccggaa cctatttccg tcggttacat gtatgtccta aagttgcacc 3240
   atctggtaga cgacaagatt catgctcgct caacaggacc gtattcgatg ataacccagc 3300
   aaccgctggg cggcaaggca caattcggtg ggcagcgctt cggagagatg gaggtgtggg 3360
   cgcttgaggc atatggtgca gcgcacgcac tccaagagtt gctaacaata aagtctgatg 3420
   acgttgttgg ccgggtaaag gtatacgatg cgattgtcaa gggttatcca attcctaccc 3480
   ccggtgtgcc tgaatcattt aaggttattg tcaaggaaat gcagtctttg tgcataaata 3540
   ttgaggttgt ttctgacggt gaggatgatg tttccgctga tgctgaaacc ctacagatag 3600
   aagaaggtct ggatacgtct cccaaggtag aagttggttc gcttgaagag gtctagtcgg 3660
   gcattcgcaa aagaggtttg ttttgtctac atcttttaaa gaaatccggg ttagtatggc 3720
   cacgactgaa gatattcgtc gttggtcata tggcgttgta a                     3761
<210> 10
   <211> 507
   <212> ADN
   <213> Tropheryma whippelii
<400> 10
   ttgagcgcac gccggaaaaa aacagtgaaa aggatctttt ttcgggcaga ataatccccg 60
   ctcgcggtgc ttggctagaa ttcgaagttg acaggcatga ccagcttggc gttagggttg 120
   acaggaagcg caggcagccg gttattttct ttctgagagc aattggcatg actgatgatg 180
   agatcaggga tgcatttggc gagtttgaat caataagcgt ccagcacgaa aagaatattg 240
   ggctgtccag agatgacgcg ctccgggaaa tataccgtcg cgttcgtccg ggggagcagg 300
   catcggctga ggctgggcgt gcactcttag agaattttta ctttaccagc agacgttttg 360
   acctggcaag ggttggaagg tacaaagtaa atcgcaaact cggtgttgat gttgatccaa 420
   ctcggatggt tcttacgagg tcggatatta tcgcaacaat tcgttatctc gcggccttgc 480
   atctcggttt ctccgaggtt gcggtgc                                     507
<210> 11
   <211> 20
   <212> ADN
   <213> Séquence artificielle
<220>
   <223> Description de la séquence artificielle:amorce
<400> 11
   ttgagcgcac gccggaaaaa 20
<210> 12
   <211> 20
   <212> ADN
   <213> Séquence artificielle
<220>
   <223> Description de la séquence artificielle:amorce
<400> 12
   gcaccgcaac ctcggagaaa 20
<210> 13
   <211> 19
   <212> ADN
   <213> Séquence artificielle
<220>
   <223> Description de la séquence artificielle:amorce
<400> 13
   cggaaacatc ccccacaat 19
<210> 14
   <211> 17
   <212> ADN
   <213> Séquence artificielle
<220>
   <223> Description de la séquence artificielle:amorce
<400> 14
   accgacgata tcgacca 17
<210> 15
   <211> 18
   <212> ADN
   <213> Séquence artificielle
<220>
   <223> Description de la séquence artificielle:amorce
<400> 15
   ttggtaaggt gaccccaa 18
<210> 16
   <211> 17
   <212> ADN
   <213> Séquence artificielle
<220>
   <223> Description de la séquence artificielle:amorce
<400> 16
   ggtaaagcgc agttcgg 17
<210> 17
   <211> 17
   <212> ADN
   <213> Séquence artificielle
<220>
   <223> Description de la séquence artificielle:amorce
<400> 17
   ccagcccgga gctggtt 17
<210> 18
   <211> 15
   <212> ADN
   <213> Séquence artificielle
<220>
   <223> Description de la séquence artificielle:amorce
<400> 18
   tttcatttgc caagc 15
<210> 19
   <211> 17
   <212> ADN
   <213> Séquence artificielle
<220>
   <223> Description de la séquence artificielle:amorce
<400> 19
   ggtaaagcgc agttcgg 17
<210> 20
   <211> 17
   <212> ADN
   <213> Séquence artificielle
<220>
   <223> Description de la séquence artificielle:amorce
<400> 20
   gtcattccaa accgtgg 17
<210> 21
   <211> 18
   <212> ADN
   <213> Séquence artificielle
<220>
   <223> Description de la séquence artificielle:amorce
<400> 21
   ctatgcacgc attagcga 18
<210> 22
   <211> 16
   <212> ADN
   <213> Séquence artificielle
<220>
   <223> Description de la séquence artificielle:amorce
<400> 22
   gaaccgcttg aggttc 16
<210> 23
   <211> 17
   <212> ADN
   <213> Séquence artificielle
<220>
   <223> Description de la séquence artificielle:amorce
<400> 23
   accttcatca tcaacgg 17
<210> 24
   <211> 16
   <212> ADN
   <213> Séquence artificielle
<220>
   <223> Description de la séquence artificielle:amorce
<400> 24
   gattcgttgc gggaca 16

## Description

La présente invention concerne le domaine du diagnostic. Plus précisément, l'invention concerne le diagnostic moléculaire de la maladie de Whipple par les techniques de détection et/ou d'amplification et séquençage à l'aide de sondes ou d'amorces oligonucléotidiques, et leur application à la recherche de la présence ou à l'identification des bactéries de l'espèce *Tropheryma whippelii.*

La maladie de Whipple est une maladie qui se présente sous des formes variées. La forme la plus classique est celle d'une fièvre avec une diarrhée chronique qui entraîne un amaigrissement, mais c'est aussi une maladie qui est susceptible de donner des atteintes articulaires chroniques, des atteintes cérébrales avec démence, des atteintes ophtalmologiques avec uvéïte et aussi une atteinte cardiaque en particulier une endocardite à hémoculture négative. Dès sa description en 1907, Whipple évoque l'existence d'une bactérie associée à la « lipodystrophie intestinale » devant l'observation de nombreux microorganismes après coloration argentique d'un ganglion mésentérique [Whipple GH (1907) Bull. John Hopkins Hosp.18 : 328]. La mise en évidence du caractère PAS (de l'anglais « periodic acid Schiff ») positif non spécifique de cette bactérie, puis les observations en microscopie électronique confirment la présence d'une espèce bactérienne intracellulaire de structure Gram positif [Chears et al. (1961) Gastroenterology 41 :1296]. L'outil moléculaire universel 16S ARNr a permis de confirmer cette hypothèse en précisant la taxonomie phylogénique de cette nouvelle espèce bactérienne, et en lui assignant le nom provisoire de *Tropheryma whippelii* pour évoquer la notion de malabsorption intestinale et honorer le découvreur de l'affection [Relman D. et al. (1992) N. Engl. J. Med. 327 :293]. Le séquençage direct de 721 bases d'un fragment amplifié à partir d'une biopsie de l'intestin grêle d'un patient [Wilson KH et al. (1991) Lancet 338 :474], puis à partir d'un ganglion d'un autre patient [Wilson KH et al. (1992) ASM News 58 :318] confirme l'originalité de l'espèce bactérienne associée à la maladie de Whipple. Le séquençage par Relman et al. (op. cité) de 1.321 bases représentant 90% du gène 16S rADN sur un échantillon, et un fragment de 284 bases chez quatre autres patients a permis de confirmer que l'espèce bactérienne associée à la maladie de Whipple représentait une nouvelle espèce, de préciser sa position taxonomique dans le phylum des actinomycètes, c'est-à-dire des bactéries de structure Gram positif à haut contenu en guanosine plus cytosine, représentant un nouvel embranchement relativement proche de deux espèces connues en pathologie humaine, *Actinomyces pyogenes* et *Rothia dentocariosa.*

Le gène *rpoB* code une des sous-unités de l'ARN polymérase bactérienne et constitue un marqueur génétique permettant la détection spécifique de la bactérie de l'espèce *Tropheryma whippelii.*

Selon Lazcano et al. [J. Mol. Evol. (1988) 27:365-376], les ARN polymérases sont divisées en deux groupes selon leur origine, l'un constitué par les ARN polymérases virales ARN- ou ADN-dépendantes, et l'autre constitué par les ARN polymérases ADN-dépendantes d'origine eucaryote ou procaryote (archaébactéries et eubactéries). Les ARN polymérases ADN-dépendantes eubactériennes sont caractérisées par une constitution multimérique simple et conservée notée « core enzyme », représentée par αββ', ou « holoenzyme » représentée par αββ'σ [Yura and Ishihama, Ann. Rev. Genet. (1979) 13 :59-97]. De nombreux travaux ont mis en évidence le rôle fonctionnel, au sein du complexe enzymatique multimérique, de la sous-unité β de l'ARN polymérase eubactérienne. Les ARN polymérases archaébactérienne et eucaryote présentent, pour leur part, une structure plus complexe pouvant atteindre une dizaine, voire une trentaine de sous-unités [Pühlet et al. Proc. Natl. Acad. Sci. USA (1989) 86 :4569-4573].

Les gènes qui codent les différentes sous-unités αββ'σ de l'ARN polymérase ADN-dépendante chez les eubactéries, respectivement les gènes *rpoA, rpoB, rpoC* et *rpoD,* sont classés en différents groupes comprenant les gènes codant pour des protéines constitutives des sous-unités ribosomiques ou pour des enzymes impliquées dans la réplication et la réparation du génome [Yura and Yshihma, Ann. Rev. Genet. (1979) 13 :59-97]. Certains auteurs ont montré que les séquences des gènes *rpoB* et *rpoC* pouvaient être utilisées afin de construire des arbres phylogénétiques [Rowland et al. Biochem. Soc. Trans. (1992) 21 :40S] permettant de séparer les différents embranchements et sous-embranchements parmi les règnes du vivant.

Le diagnostic de la maladie est actuellement fait par l'observation, après coloration, de frottis microscopiques obtenus de biopsie ou par amplification et séquençage de l'outil gène universel 16S ARNr (Relman et al., op. cité).

Toutefois, le gène 16S est un gène moyennement discriminant pour l'identification des bactéries. En outre, il est nécessaire de disposer de plusieurs cibles moléculaires d'identification des bactéries, notamment pour pallier les problèmes de contamination moléculaire.

Par ailleurs, cette bactérie a été pour la première fois isolée et cultivée dans des systèmes cellulaires dans le laboratoire des inventeurs [Raoult D. et al. (2000) N. Engl. J. Med. 342 :620 et WO 00/58440] à partir de la valve cardiaque prélevée chez un patient présentant une endocardite à hémoculture négative (souche Twist).

Les inventeurs ont déjà décrit dans WO - A - 00/58440 un fragment de 612 bases du gène *rpoB* de la bactérie *Thropheryma whippelii* correspondant à SEQ ID N° 3 à partir duquel ils avaient déterminé deux séquences nucléotidiques spécifiques du gène *rpoB* de *Thropheryma whippelii* utilisables comme amorces d'amplification spécifiques et sondes de détection spécifiques de l'espèce *Tropheryma whippelii.*

Les techniques décrites dans WO - A - 00/58440 n'avaient pourtant pas permis d'obtenir la séquence complète du gène *rpoB* de *Tropheryma whippelii.*

Un but de la présente invention est de fournir des fragments du gène *rpoB* additionnels, notamment la séquence complète du gène *rpoB* de *Tropheryma whippelii,* afin en particulier de fournir de nouvelles séquences spécifiques du gène *rpoB* de *Tropheryma whippelii;* plus particulièrement encore, des séquences présentant une plus grande spécificité de détection que les séquences tirées des fragments SEQ ID N°3 décrites dans WO - A - 00/58440.

Plusieurs tentatives pour allonger la séquence SEQ ID N° 3 avec des amorces consensus issues de gène *rpoB* d'autres bactéries et des amorces spécifiques développées par les inventeurs (SEQ ID N°13, 15 et 17) n'ont permis d'allonger la séquence que jusqu'à l'obtention de la séquence SEQ ID N°4 de 2750 Pb décrites ci-après, mais n'ont pas ensuite permis d'obtenir la séquence complète du gène. Les inventeurs ont développé une série d'amorces spécifiques pour mettre en oeuvre la technique dite « marche sur le génome » (« Genome Walker » décrite dans Siebert et al Nucleic Acids Research 23 :1087-1088, 1995) à partir de la détermination d'une série d'amorces d'amplification spécifique, à savoir les amorces d'amplification identifiées ci-après comme SEQ ID N° 5 à 8. Ces différentes amorces d'amplification ont permis de proche en proche d'obtenir des fragments de plus en plus étendus à partir de SEQ ID N° 4, jusqu'à obtenir la séquence complète SED ID N° 9.

La présente invention a donc pour objet la séquence complète du gène *rpoB* de la bactérie *Tropheryma whippelii* constituée par la séquence SEQ ID N° 9. La séquence complète du gène *rpoB* a été déterminée par la combinaison de deux techniques, l'amplification enzymatique et le séquençage automatique direct avec des amorces consensus entre un grand nombre d'autres bactéries, de genre et d'espèces différentes d'une part ; et par séquençage automatique direct à partir des produits d'amplification obtenus après application de la technique Genome Walker (marche sur le génome) à l'ADN extrait de la première souche Twist de *Tropheryma whippelii,* d'autre part. Cette dernière technique consiste à marcher le long du gène à partir d'un premier fragment connu en réalisant une banque partielle de l'ADN chromosomique de la bactérie étudiée. Cette technique, qui sera décrite plus loin, requiert donc une quantité d'ADN chromosomique bactérien qui ne peut être obtenue qu'après extraction dudit ADN à partir d'une culture bactérienne pure et elle suppose donc la disponibilité d'un isolat en culture. C'est la disponibilité de cet isolat et la détermination des différentes amorces d'amplification SEQ ID N° 5 à 8 à partir de SEQ ID N° 4 qui ont permis l'application avec succès de la technique de marche sur le génome pour la détermination de la séquence complète du gène *rpoB* chez une souche Twist de la bactérie *Tropheryma whippelii.*

La présente invention concerne donc également des séquences d'acides nucléiques de l'espèce *Trophyrema whippelii,* dont la séquence nucléotidique est comprise dans la séquence SEQ ID N° 9 du gène *rpoB* de ladite bactérie visée ci-dessus et non entièrement comprise dans la SEQ ID N° 3, ou une séquence complémentaire de ladite séquence nucléotidique.

A partir de la séquence complète SEQ ID N° 9, les inventeurs ont en effet déterminé une séquence SEQ ID N° 10 de plus grande spécificité vis à vis du gène *rpoB* de *Tropheryma whippelii* en dehors de la zone correspondant à SEO ID N° 3, à partir de laquelle ils ont pu déterminer deux séquences particulières spécifiques encadrant un fragment d'environ 500 paires de bases, à savoir les séquences SEQ ID N° 11 et 12 conférant une fiabilité optimale pour la détection spécifique de la bactérie *Tropheryma whippelii* par amplification et séquençage du produit d'amplification ou par hybridation en tant que sonde de détection.

Un objet de la présente invention est donc un oligonucléotide monocaténaire choisi parmi les oligonucléotides ayant une séquence d'au moins 10 motifs nucléotidiques consécutifs incluse dans une séquence choisie parmi :
- la séquence SEQ ID N° 9 et non entièrement comprise dans la séquence SEO ID N° 3 et de préférence non comprise dans SEQ ID N° 3, et
- de préférence l'une des séquences SEQ ID N°10, SED ID N° 11 et SEQ ID N°12, et
- les séquences complémentaires de ces oligonucléotides.

Un oligonucléotide plus particulièrement préféré a une séquence choisie parmi les séquences complètes SEQ ID N°11 et SEQ ID n°12 et leurs séquences complémentaires.

La présente invention a également pour objet un oligonucléotide qui présente une séquence choisie parmi :
- les séquences d'au moins 10 nucléotides consécutifs incluses dans une séquence choisie par les séquences SEQ ID N° 5 à 8 et SEQ ID N° 17, et leurs séquences complémentaires, et
- de préférence, lesdites séquences SEQ ID N° 5 à 8 et SEQ ID N° 17 complètes et leurs séquences complémentaires.

En particulier les oligonucléotides selon la présente invention possèdent au moins 10 motifs nucléotidiques tels que décrits ci-dessus et au plus 100, de préférence au plus 50 motifs. Plus particulièrement, un oligonucléotide selon la présente invention possède de préférence de 12 à 35 motifs, de préférence encore de 18 à 22.

Les séquences nucléotidiques selon l'invention peuvent être préparées par synthèse chimique en utilisant les techniques bien connues de l'homme du métier et décrites par exemple dans l'article de Itakura K. et al. [(1984) Annu. Rev. Biochem. 53 : 323].

Une première application d'un oligonucléotide selon l'invention est son utilisation comme sonde d'espèce pour la détection, dans un échantillon biologique, de bactéries de l'espèce *Tropheryma whippelii.* Dans la suite de la description, une telle sonde de l'invention sera appelée sonde d'espèce.

De préférence une sonde d'espèce selon l'invention est constituée par un oligonucléotide choisi parmi :
- les oligonucléotides ayant une séquence nucléotidique d'au moins 10 nucléotides consécutifs incluse dans l'une des séquences SEQ ID N° 10, SEQ ID N° 11 et SEQ ID N° 12, et leurs séquences complémentaires, et
- les oligonucléotides ayant une séquence nucléotidiques choisies parmi les séquences complètes SEQ ID N° 11 et SEQ ID N° 12 et leurs séquences complémentaires.

Les sondes selon l'invention peuvent être utilisées, à des fins de diagnostic, dans la recherche de la présence ou de l'absence d'un acide nucléique cible dans un échantillon, selon toutes les techniques d'hybridation connues et notamment les techniques de dépôt ponctuel sur filtre, dites « DOT-BLOT » [Maniatis et al. (1982) Molecular Cloning, Cold Spring Harbor], les techniques de transfert d'ADN dites « SOUTHERN BLOT » [Southern E.M., J. Mol. Biol. (1975) 98 :503], les techniques de transfert d'ARN dites « NORTHERN BLOT », ou les techniques dites « sandwich », avec une sonde de capture et/ou une sonde de détection, lesdites sondes étant capables de s'hybrider avec deux régions différentes de l'acide nucléique cible, et l'une au moins desdites sondes (généralement la sonde de détection) étant capable de s'hybrider avec une région de la cible qui est spécifique de l'espèce, étant entendu que la sonde de capture et la sonde de détection doivent avoir des séquences nucléotidiques au moins partiellement différentes.

L'acide nucléique à détecter (cible) peut être obtenu suite à une amplification classique, c'est à dire donnant de l'ADN, obtenu après amplification par PCR, ou de l'ARN, obtenu suite à une amplification transcriptionnelle par TAM, NASBA, 3SR etc... Dans le cas de la détection d'une cible de type acide nucléique double brin, il convient de procéder à la dénaturation de ce dernier avant la mise en oeuvre du procédé de détection. L'acide nucléique cible peut être obtenu par extraction selon les méthodes connues des acides nucléiques d'un échantillon à examiner. La dénaturation d'un acide nucléique double brin peut être effectuée par les méthodes connues de dénaturation chimique, physique ou enzymatique, et en particulier par chauffage à une température appropriée, supérieure à 80°C.

Pour mettre en oeuvre les techniques d'hybridation précitées, et en particulier les techniques « sandwich », une sonde de l'invention, appelée sonde de capture est immobilisée sur un support solide, et une autre sonde de l'invention, appelée sonde de détection, est marquée avec un agent marqueur. Les exemples de support et d'agent marqueur sont tels que définis précédemment.

Un autre objet de l'invention est un procédé de détermination de la présence ou de l'absence d'une bactérie *Tropheryma whippelii* dans un échantillon contenant ou susceptible de contenir des acides nucléiques d'au moins une telle bactérie, comprenant les étapes consistant à mettre en contact ledit échantillon avec au moins une sonde d'espèce de l'invention, puis à déterminer de façon connue en soi la formation ou l'absence de formation d'un complexe d'hybridation entre ladite sonde et l'acide nucléique de l'échantillon.

Des exemples de détection de la formation ou de l'absence de formation d'un complexe d'hybridation entre ladite sonde et l'acide nucléique comprennent les techniques « DOT-BLOT », « SOUTHERN-BLOT » et « sandwich ».

Selon une mise en oeuvre particulière de ce procédé pour la détermination de la présence ou de l'absence d'une espèce *Tropheryma whippelii,* on utilise plusieurs sondes d'espèce de l'invention, étant entendu que lesdites sondes sont capables de s'hybrider avec des régions non chevauchantes d'un acide nucléique correspondant au gène *rpoB* de *Tropheryma whippelii.*

De manière avantageuse, une sonde d'espèce est immobilisée sur un support solide, et une autre sonde est marquée par un agent marqueur, dite sonde de détection, laquelle n'est pas nécessairement une sonde d'espèce.

Une autre application d'un oligonucléotide de l'invention est son utilisation comme amorce nucléotidique utilisable dans la synthèse d'un acide nucléique de *Thropheryma whippelii* en présence d'une polymérase par un procédé connu en soi, notamment dans des méthodes d'amplification utilisant une telle synthèse en présence d'une polymérase (PCR, RT-PCR, etc...). En particulier, une amorce de l'invention peut être utilisée pour la transcription inverse spécifique d'une séquence d'ARN messager de *Tropheryma whippelii* pour obtenir une séquence d'ADN complémentaire correspondante. Une telle transcription inverse peut constituer le premier stade de la technique RT-PCR, le stade suivant étant l'amplification par PCR de l'ADN complémentaire obtenu. On peut également utiliser les amorces de l'invention pour l'amplification spécifique par réaction de polymérisation en chaîne de la séquence totale de l'ADN du gène *rpoB* de *Tropheryma whippelii.*

Selon un cas particulier ladite amorce comprenant un oligonucléotide de l'invention comprend en outre la séquence sens ou anti-sens d'un promoteur reconnu par une ARN polymérase (promoteurs T7, T3, SP6 par exemple [Studier FW, BA Moffatt (1986) J. Mol. Biol. 189 :113] : de telles amorces sont utilisables dans des procédés d'amplification d'acide nucléique faisant intervenir une étape de transcription, tels que, par exemple, les techniques NASBA ou 3SR [Van Gemen B. et al. Abstract MA 1091, 7th International Conference on AIDS (1991) Florence, Italy].

Plus particulièrement, un objet de l'invention est une amorce nucléotidique utilisable pour le séquençage total ou partiel du gène *rpoB* d'une souche quelconque de *Tropheryma whippelii,* de préférence une séquence spécifique du gène *rpoB* de *Thropheryma whippelii.* En particulier, l'amorce nucléotidique est utilisable pour le séquençage d'un acide nucléique amplifié. Le séquençage est l'obtention de la séquence totale ou partielle du gène *rpoB* par un procédé connu en soi, polymérisation abortive utilisant les di-déoxynucléotides [Sanger F., Coulson AR (1975) J. Mol. Biol. 94:441] ou hybridations multiples utilisant les puces à ADN.

De préférence, dans une utilisation comme amorce pour le séquençage d'une séquence spécifique du gène *rpoB de Thropheryma whippelii,* on utilise un oligonucléotide qui est constitué par :
- les oligonucléotides ayant une séquence nucléotidique d'au moins 10 nucléotides consécutifs incluses dans l'une des séquences SEQ ID N° 10, SEQ ID N° 11 et SEQ ID N° 12, et leurs séquences complémentaires, et
- les oligonucléotides ayant une séquence nucléotidiques choisies parmi les séquences complètes SEQ ID N° 11 et SEQ ID N° 12 et leurs séquences complémentaires.

La présente invention fournit donc également un procédé de détermination de la présence ou de l'absence d'une bactérie *Tropheryma whippelii* dans un échantillon contenant, ou susceptible de contenir des acides nucléiques d'au moins une telle bactérie, caractérisé en ce qu'on réalise une amplification de l'ADN de l'échantillon à l'aide de deux amorces selon l'invention et on compare la séquence obtenue avec la séquence SEQ ID N°9.

Plus particulièrement, on compare la séquence du produit d'amplification obtenu en réalisant au préalable son séquençage.

De préférence, on réalise une amplification de l'ADN de l'échantillon à l'aide des deux amorces SEQ ID N° 11 et 12 et on compare la séquence obtenue avec la séquence SEQ ID N° 10.

L'invention sera mieux comprise à l'aide de l'exposé ci-après, divisé en deux exemples, qui concernent des expériences effectuées dans le but de réaliser l'invention et qui sont données à titre purement illustratif et explicatif.

La figure n°1 représente les positions des amorces spécifiques et consensuelles utilisées pour la détermination des fragments successifs du gène complet *rpoB* de l'espèce bactérienne *Tropheryma whippelii.*

La figure 2 représente la visualisation des produits d'amplification obtenus à l'aide des amorces SEQ ID N° 11 et 12 visualisés par coloration au bromure d'éthidium après électrophorèse sur un gel d'agarose de l'exemple 2.

Différents termes, utilisés dans la description et les revendications, sont définis ci-après:
- par « acide nucléique extrait de bactéries » on entend soit l'acide nucléique total, soit l'ADN génomique, soit les ARN messagers, soit encore l'ADN obtenu à partir de la transcription inverse des ARN messagers, soit encore les ARN ribosomaux ;
- un « fragment nucléotidique » ou un « oligonucléotide » sont deux termes synonymes désignant un enchaînement de motifs nucléotidiques caractérisé par une séquence informationnelle des acides nucléiques naturels (ou éventuellement modifiés) et susceptibles de s'hybrider, comme les acides nucléiques naturels, avec un fragment nucléotidique complémentaire ou sensiblement complémentaire, dans des conditions prédéterminées de stringence stricte. L'enchaînement peut contenir des motifs nucléotidiques de structure différente de celle des acides nucléiques naturels. Un fragment nucléotidique (ou oligonucléotide) peut contenir par exemple jusqu'à 100 motifs nucléotidiques. Il contient généralement au moins 10, et en particulier au moins 12 motifs nucléotidiques et peut être obtenu à partir d'une molécule d'acide nucléique naturelle et/ou par recombinaison génétique et/ou par synthèse chimique,
- un motif nucléotidique est dérivé d'un monomère qui peut être un nucléotide naturel d'acide nucléique dont les éléments constitutifs sont un sucre, un groupement phosphate et une base azotée choisie parmi l'adénine, la guanine, l'uracile, la cytosine, la thymine ; ou bien le monomère est un nucléotide modifié dans l'un au moins des trois éléments constitutifs précédents ; à titre d'exemple, la modification peut intervenir soit au niveau des bases, avec des bases modifiées telles que l'inosine, la méthyl-5-désoxycytidine, la désoxyuridine, la diméthylamino-5-désoxyuridine ou toute autre base modifiée capable d'hybridation, soit au niveau du sucre, par exemple le remplacement d'au moins un désoxyribose par un polyamide [Nielsen PE et al., Science (1991) 254 :1497-1500], soit encore au niveau du groupement phosphate, par exemple par remplacement par des esters choisis notamment parmi les diphosphates, les alkylphosphonates et les phosphorothioates,
- par « hybridation », on entend le processus au cours duquel, dans des conditions appropriées, deux fragments nucléotidiques ayant des séquences suffisamment complémentaires sont susceptibles de s'associer par des liaisons hydrogène stables et spécifiques, pour former un double brin. Les conditions d'hybridation sont déterminées par la « stringence », c'est-à-dire la rigueur des conditions opératoires. L'hybridation est d'autant plus spécifique qu'elle est effectuée à plus forte stringence. La stringence est fonction notamment de la composition en bases d'un duplex sonde/cible, ainsi que par le degré de mésappariement entre deux acides nucléiques. La stringence peut également être fonction des paramètres de la réaction d'hybridation, tels que la concentration et le type d'espèces ioniques présentes dans la solution d'hybridation, la nature et la concentration d'agents dénaturants et/ou la température d'hybridation. La stringence des conditions dans lesquelles une réaction d 'hybridation doit être réalisée dépend notamment des sondes utilisées. Toutes ces données sont bien connues et les conditions appropriées peuvent éventuellement être déterminées dans chaque cas par des expériences de routine. En général, selon la longueur des sondes utilisées, la température pour la réaction d'hybridation est comprise entre environ 20 et 65°C, en particulier entre 35 et 65°C dans une solution saline à une concentration d'environ 0,8 à 1 M.
- une « sonde » est un fragment nucléotidique comprenant par exemple de 10 à 100 motifs nucléotidiques, notamment de 12 à 35 motifs nucélotidiques, possédant une spécificité d'hybridation dans des conditions déterminées pour former un complexe d 'hybridation avec un acide nucléique ayant, dans le cas présent, une séquence nucléotidique comprise soit dans un ARN messager, soit dans un ADN obtenu par transcription inverse dudit ARN messager, produit de transcription ; une sonde peut être utilisée à des fins de diagnostic (notamment sondes de capture ou de détection) ou à des fins de thérapie,
- une « sonde de capture » est immobilisée ou immobilisable sur un support solide par tout moyen approprié, par exemple par covalence, par adsorption, ou par synthèse directe sur un solide. Des exemples de supports comprennent les plaques de microtitration et les puces à ADN, ou des particules magnétiques, comme celles décrites dans WO 6 A - 99/35500.
- une « sonde de détection » peut être marquée au moyen d'un agent marqueur choisi par exemple parmi les isotopes radioactifs, les enzymes, en particulier les enzymes susceptibles d'agir sur un substrat chromogéne, fluorigène ou luminescent (notamment une peroxydase ou une phosphatase alcaline), les composés chimiques chromophores, les composés chromogènes, fluorigènes ou luminescents, les analogues des bases nucléotidiques et les ligands tels que la biotine,
- une « sonde d'espèce » est une sonde permettant l'identification de l'espèce d'une bactérie,
- une « amorce » est une séquence comprenant par exemple 10 à 100 motifs nucléotidiques et possédant une spécificité d'hybridation dans des conditions déterminées pour l'initiation d'une polymérisation enzymatique, par exemple dans une technique d'amplification telle que la PCR, dans un procédé de séquençage, dans une méthode de transcription, etc.

### EXEMPLE 1 : Séquence complète du gène rpoB de Tropheryma whippelii.

La séquence complète du gène *rpoB* de *Tropheryma whippelii* a été déterminée par la combinaison successive de deux techniques, l'amplification enzymatique et séquençage automatique direct utilisant des amorces consensus dans un premier temps puis la marche sur le gène par la technique du « Genome Walker » dans un deuxième temps.

### 1 - Amplification enzymatique associée au séquençage automatique

Les amorces consensus ayant permis l'obtention d'un premier fragment de la séquence *Tropheryma whippelii rpoB* ont été déterminées à partir de la comparaison des séquences *rpoB* de *Bacillus subtilis, Bartonella henselae, Borrelia burgdorferi, Buchnera aphidicola, Chlamydia pneumoniae, Chlamydia trachomatis, Coxiella bumetii, Escherichia coli, Haemophilus influenzae, Helicobacter pylori, Legionella pneumophila, Mycobacterium leprae, Mycobacterium smegmatis, Mycobacterium tuberculosis, Mycoplasma gallisepticum, Mycoplasma genitalium, Mycoplasma pneumoniae, Neisseria meningitidis, Pseudomonas putida, Rickettsia prowazekii, Rickettsia typhi, Salmonella enterica* Typhimurium, *Spiroplasma citri, Staphylococcus aureus, Synechocystis* spp., *Thermotoga maritima, Treponema pallidum,* et Human Granulocytic Ehrlichiosis agent. Ces séquences ont été choisies par alignement des séquences de l'ADN du gène *rpoB* de ces différentes autres bactéries et en sélectionnant celles qui étaient les plus conservées avec pour hypothèse qu'elles étaient aussi présentes dans le gène *rpoB* de *Tropheryma whippelii.* Les amorces utilisées avaient pour séquence :
SEQ ID n°1 = 5' - TIA TGG GII CIA AIA TGC A- 3' (position 1967-1985)
SEQ ID n°2 = 5'- GCC CAI CAT TCC ATI TCI CC - 3' (position 3198-3217, reverse), dans lesquelles I représente l'inosine.

La numérotation des positions mentionnées ci-dessus et ci-après correspondent à des positions sur la séquence complète SEQ ID N°9.

Ces amorces ont permis l'amplification puis le séquençage d'un fragment de 1.400 paires de bases dont 612 paires de bases ont été séquencées à l'aide des amorces SEQ ID n°1 et SEQ ID n°2 et correspond à la séquence SEQ ID n°3 (positions 2063-2673) dont la séquence est :

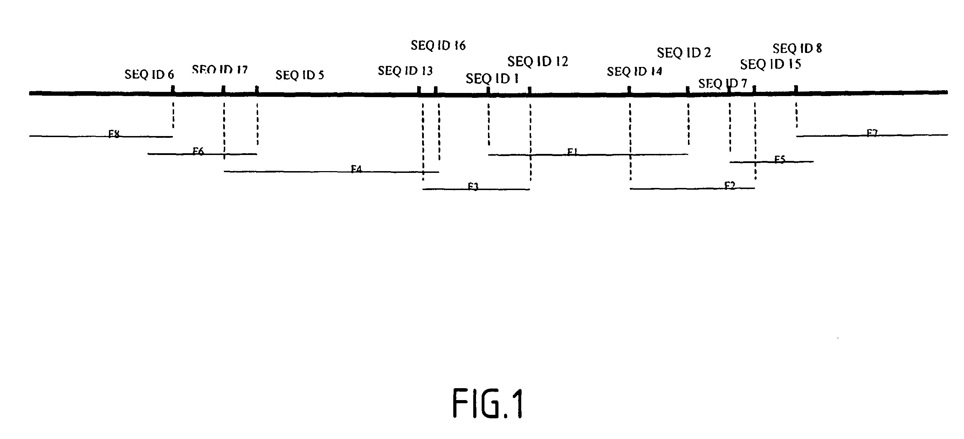

Le séquençage de ce premier fragment F1 a permis de déterminer des amorces spécifiques d'amplification qui, combinées dans des réactions d'amplification PCR avec des amorces consensuelles dégénérées, ont permis d'allonger la séquence SEQ ID n°3 sur ses extrémités 3' et 5' et ont permis d'obtenir trois autres fragments chevauchant F2, F3 et F4 pour une longueur totale de 2.750 paires de bases correspondant à SEQ ID N° 4. La position des amorces et des fragments est indiquée sur la figure n°1. Le fragment F3 (positions 1248 à 2163) a été obtenu par application des amorces SEQ ID N°13 (amorce spécifique ; positions 2145 à 2163) : 5' - CGG AAA CAT CCC CCA CAA T - 3' et SEQ ID N°14 (amorce consensuelle ; positions 1248 à 1264) : 5' - ACC GAC GAT ATC GAC CA - 3' ; le fragment F2 (positions 2592 à 3229) a été obtenu par application des amorces SEQ ID N°15 (amorce spécifique ; positions 2592 à 2610) : 5' - TTG GTA AGG TGA CCC CAA A -3' et SEQ ID N°16 (amorce consensuelle ; positions 3213 à 3229) : 5' - GGT AAA GCG CAG TTC GG - 3' ; le fragment F4 (positions 484 à 1331) a été obtenu par application des amorces SEQ ID N°17 (amorce spécifique ; positions 1315 à 1331) : 5' - CCA GCC CGG AGC TGG TT - 3' et SEQ ID N°18 (amorce consensuelle ; positions 484 à 498) : 5' - TTT CAT TTG CCA AGC - 3'.

Les tentatives suivantes pour allonger cette séquence par la technique des amorces consensus ont ensuite échoué, ne permettant pas de poursuivre le séquençage du gène selon cette approche. En particulier, les amorces consensus suivantes choisies parmi les séquences les plus conservées des gènes *rpoB* d'autres bactéries phylogénétiquement proches de *Tropheryma whippelii: Mycobacterium smegmatis, Mycobacterium leprae, Mycobacterium tuberculosis, Bacillus licheniformis, Salmonella enterica* Typhimurium ont été testées sans succès : Balich7f : 5' - GGT AAA GCG CAG TTC GG - 3' (SEQ ID N° 19) ; Balich 460f : 5' - GTC ATT CCA AAC CGT GG - 3' (SEQ ID N° 20) ; Balich37f : 5' - CTA TGC ACG CAT TAG CGA - 3'(SEQ ID N° 21) ; Myco12F : 5' - GAA CCG CTT GAG GTT C - 3'(SEQ ID N° 22) ; Myco43F : 5'- ACC TTC ATC ATC AAC GG - 3'(SEQ ID N° 23) ; Myco8R : 5' - GAT TCG TTG CGG GAC A - 3'(SEQ ID N° 24).

### 2 - Marche sur le gène par la technique du « Genome Walker »

Cet échec survenant sur la souche de *Tropheryma whippelii* en culture explique qu'il aurait été impossible d'obtenir a fortiori la totalité de la séquence du gène *rpoB* de *Tropheryma whippelii* à partir de prélèvements cliniques contenant la bactérie *Tropheryma whippelii.* Une deuxième technique dite de "marche sur le génome » a été ensuite appliquée afin de compléter la séquence de *Tropheryma whippelii rpoB* dans la direction 3' et dans la direction 5'. Cette technique a été décrite [(1995) Siebert et al. Nucl. Acids Res.23 :1087-1088] et consiste à réaliser une banque de l'ADN génomique de la bactérie puis à amplifier des fragments de cette banque à l'aide d'amorces universelles indépendantes de la séquence recherchée et d'amorces spécifiques de la séquence recherchée. Après avoir extrait une grande quantité d'ADN (de l'ordre du microgramme) purifié à partir de la souche bactérienne *Tropheryma whippelii* Twist décrite dans WO 00/58440, l'ADN bactérien est fragmenté par digestion enzymatique à l'aide d'endonucléases de restriction et les fragments ainsi obtenus sont liés à l'une de leurs deux extrémités à des oligonucléotides synthétiques appelés adaptateurs fournis dans une trousse commercialisée (Genome Walker kit^{®}, Clontech Laboratories, Palo Alto, Californie). Des oligonucléotides complémentaires des adaptateurs, également fournis dans la même trousse de laboratoire, constituent alors des amorces d'amplification pour la technique d'amplification enzymatique en chaîne (« polymerase chain reaction », PCR). Ces oligonucléotides constituent donc des amorces universelles qui sont utilisées dans cette technique indépendamment de la séquence de l'ADN bactérien étudié. Afin de réaliser les réactions de PCR, un deuxième oligonucléotide doit être incorporé comme amorce d'amplification dans les réactions PCR. Cet oligonucléotide amorce doit répondre à plusieurs critères et la détermination de sa séquence constitue donc un aspect de l'invention : (1) la séquence de cet oligonucléotide est complémentaire de SEQ ID n°4 (2) cet oligonucléotide est positionné à une cinquantaine de bases de l'extrémité de SEQ ID n°4 (3) cet oligonucléotide a une longueur supérieure à 20 bases. L'utilisation d'un premier oligonucéotide présentant ces caractéristiques correspondant à la séquence SEQ ID n° 5 = 5'-CTT ATC TCG CCC GTA TCA TG - 3' (position 627-646, inverse) a ainsi permis d'obtenir un fragment F6 (positions 146 à 646) de 500 paires de bases. Sur la base de la séquence ainsi obtenue, l'utilisation d'un second oligonucléotide spécifique correspondant à la séquence SEQ ID N° 6 : = 5' - CCA GTC AAA ACT ATC GAG CTG CAA A - 3' (position 307-331, inverse) a permis l'amplification d'un fragment F8 de 1.100 paires de bases contenant l'extrémité 3' du gène *rpoB* (positions 1 à 331) et s'étendant en aval de cette extrémité. Par ailleurs, sur la base de la séquence SEQ ID N°4, on a allongé la séquence SEQ ID N°4 dans le sens 5' par l'utilisation d'un oligonucléotide SEQ ID N° 7 : 5' - TTC TCT ATG ATG GCC GCA C -3' (position 3168-3187) d'un fragment F5 (positions 3168 à 3668) de 500 paires de bases. Enfin, sur la base de ce dernier fragment, un oligonucléotide spécifique a été déterminé de séquence SEQ ID N° 8 : 5' - GCA GCG CTT CGG AGA GAT GGA G - 3' (position 3357-3379) qui a permis d'obtenir un fragment F7 de 1.500 paires de bases contenant l'extrémité 5' du gène *rpoB* (positions 3357 à 3657) et s'étendant au delà de cette extrémité. Cette technique de marche sur le génome suppose l'obtention d'une grande quantité d'ADN chromosomique de *Tropheryma whippelii* et son application a été rendue possible par l'obtention de la souche Twist de la bactérie *Tropheryma whipelii* et l'extraction en grande quantité d'ADN à partir de ladite souche. L'obtention d'une souche isolée et cultivée de *Tropheryma whippelii* était nécessaire pour la détermination de la séquence entière de *rpoB* chez la bactérie *Tropheryma whippelii.*

L'amplification de la banque génomique à l'aide des oligonucléotides SEQ N° 5 à SEQ N° 8 a permis d'allonger dans les sens 3' et 5' la séquence SEQ ID n°4 afin d'obtenir la séquence complète du gène *rpoB* de *Tropheryma whippelii* souche Twist. La séquence complète du gène *rpoB* de *Tropheryma whippelii* obtenue par application des techniques d'amplification consensuelle et de marche sur le génome déposée dans GenBank avec comme numéro d'accès (accession number) AF243072, correspond à SEQ ID n° 9. Au total, cette séquence SEQ ID N°9 a été obtenue selon le schéma de la figure n°1 qui précise la position des amorces consensuelles et des fragments successifs obtenus :
Dans la séquence SEQ ID N° 9 représentée ci-après, la séquence SEQ ID N° 4 est soulignée, et la séquence en caractères gras sont dans l'ordre :
   - SEQ ID N° 6 (séquence complémentaire),
   - SEQ ID N° 5 (séquence complémentaire),
   - SEQ ID N° 11 (séquence identique),
   - SEQ ID N° 12 (séquence complémentaire),
   - SEQ ID N° 7 (séquence identique),
   - SEQ ID N° 8 (séquence identique),

Cette séquence mesure 3.657 paires de bases et présente un contenu en cytosine plus guanosine de 50.4%.

### Exemple 2 : Détection spécifique du gène rpoB de Tropheryma whippelii.

Deux amorces spécifiques ont été déterminées à partir de la SEQ ID n°9 de façon à encadrer de façon spécifique un fragment de 507 paires de bases du gène rpoB de Tropheryma whippelii, de séquence SEQ ID N° 10 = 5' - **TTGAGCGCACGCCGGAAAAA**AACAGTGAAAAGGATCTTTTTTCGGGCAGAAT AATCCCCGCTCGCGGTGCTTGGCTAGAATTCGAAGTTGACAGGCATGACCAG CTTGGCGTTAGGGTTGACAGGAAGCGCAGGCAGCCGGTTATTTTCTTTCTGA GAGCAATTGGCATGACTGATGATGAGATCAGGGATGCATTTGGCGAGTTTGAA TCAATAAGCGTCCAGCACGAAAAGAATATTGGGCTGTCCAGAGATGACGCGC TCCGGGAAATATACCGTCGCGTTCGTCCGGGGGAGCAGGCATCGGCTGAGG CTGGGCGTGCACTCTTAGAGAATTTTTACTTTACCAGCAGACGTTTTGACCTG GCAAGGGTTGGAAGGTACAAAGTAAATCGCAAACTCGGTGTTGATGTTGATC CAACTCGGATGGTTCTTACGAGGTCGGATATTATCGCAACAATTCGTTATCTCG CGGCCTTGCATCTCGGTTTCTCCGAGGTTGCGGTGC - 3'

La détermination de ces deux amorces SEQ ID N° 11 et SEQ ID N° 12 a été réalisée in silico de façon à respecter des contraintes propres aux amorces d'identification moléculaire. L'énoncé de ces contraintes fait partie intégrante du processus inventif en ce qu'il n'est pas disponible actuellement dans la littérature scientifique: (1) longueur de 18 - 22 paires de bases (2) séquences des deux amorces présentant une température de fusion voisine (3) séquences des deux amorces ne permettant pas d'auto-hybridation ni de complémentarité (4) séquence encadrée par ces deux amorces spécifique de la bactérie *Tropheryma whippelii;* cette condition est vérifiée après alignement de la SEQ ID n°9 avec l'ensemble des séquences des gènes bactériens *rpoB* disponibles dans les banques de données informatiques ; (5) séquence encadrée par ces deux amorces présentant une longueur optimale d'environ 500 paires de bases ; cette longueur optimale correspondant à un compromis entre la recherche d'une région d'identification la plus longue possible permettant donc une discrimination la plus grande possible entre une séquence quelconque issue de la SEQ n°9 et l'ensemble des séquences *rpoB* bactériennes homologues connues et la recherche d'une région d'identification la plus courte possible afin d'augmenter le plus possible la sensibilité de la détection moléculaire. Il est en effet acquis qu'il existe une relation inverse entre sensibilité de détection et longueur de la séquence d'ADN bactérien à détecter dans un prélèvement. Enfin, la taille de 500 paires de bases correspond à la longueur moyenne de séquence obtenue après un run sur un séquenceur automatique actuellement disponible. Ces deux amorces sont localisées respectivement en position 713-745 et 1181-1200 de la séquence SEQ ID N° 9 du gène *rpoB* de la bactérie *Tropheryma whippelii.* La séquence de ces deux amorces est la suivante :
SEQ ID n°11 = 5'- TTG AGC GCA CGC CGG AAA AA - 3'
SEQ ID n°12 = 5'- GCA CCG CAA CCT CGG AGA AA - 3' (amorce inverse complémentaire)

La région du gène *rpoB* de *Tropheryma whippelii* encadrée par ces deux amorces, région dénommée SEQ ID N° 10 (position 713-1200) présente le plus haut taux de discrimination à savoir un pourcentage d'homologie inférieur à 70% par rapport aux autres régions du même gène, y compris par rapport à la région SEQ ID N° 3 présentant un pourcentage d'homologie de 75 à 85 %.

Il est important de maintenir le taux de discrimination le plus haut afin de tenir compte de la marge d'erreur introduite lors des manipulations des échantillons en pratique de laboratoire diagnostique et qui résultent des erreurs d'amplification commises par les polymérases thermostables, en particulier la *Taq* polymérase et des erreurs de séquençage. Les erreurs ainsi introduites sont de l'ordre de 0.5%.

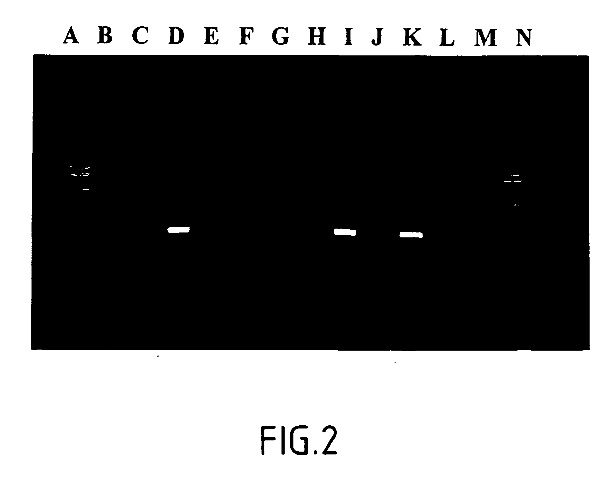

Le gène *rpoB* a été amplifié par la technique PCR utilisant 35 cycles d'amplification comportant chacun une phase de dénaturation de 94°C pendant 30 secondes, une phase d'hybridation des amorces SEQ ID n° 11 et 12 à 58°C pendant 30 secondes et une phase d'élongation à 72°C pendant 60 secondes. Le produit d'amplification est visualisé après coloration par le bromure d'éthidium (Figure 2). Les bactéries contrôles : *Mycobacterium avium, Mycobacterium tuberculosis, Nocardia otitidiscaviarum, Escherichia coli, Staphylococcus epidermidis, Corynebacterium amycolatum* n'ont pas été amplifiées par les oligonucléotides amorces SEQ ID n°11 et 12, démontrant la spécificité des ces amorces sur des espèces bactériennes phylogénétiquement proches de *Tropheryma whippelii.* Cette technique a été appliquée à la détection de la présence et de l'absence du gène *rpoB* de *Tropheryma whippelii* dans dix biopsies jéjunales obtenues chez dix patients différents dont trois biopsies avaient auparavant été montrées contenir de l'ADN de *Tropheryma whippelii* de façon indépendante et dont sept avaient été montrées exemptes d'ADN de *Tropheryma whippelii.* Cette technique a été appliquée de façon aveugle après codage des dix prélèvements, les expérimentateurs ne connaissant pas le code et ne connaissant donc pas la qualité des prélèvements travaillés. Cette technique a permis de détecter 100% de vrais positifs et 100% de vrais négatifs. Les produits d'amplification obtenus pour les trois biopsies positives ont été séquencés et les trois séquences obtenues avaient 100% d'homologie avec SEQ ID n° 10.

Sur la figure 2 sont représentés les produits d'amplification PCR obtenus à partir de dix biopsies jéjunales prélevées chez des patients présentant une maladie de Whipple confirmée (colonnes D, I et K) et ne présentant pas une maladie de Whipple (colonnes C, E, F, G, H, J, et L). Les colonnes A et N représentent le marqueur de poids moléculaire, les colonnes B et M correspondent aux témoins négatifs d'amplification (eau stérile). Les produits d'amplification sont obtenus après incorporation des amorces SEQ ID n°11 et n°12 selon l'invention et sont visualisés par coloration au bromure d'éthidium après électrophorèse sur un gel d'agarose.

## Revendications

1. Séquence totale du gène *rpoB* de la bactérie *Tropheryma whippelii,* **caractérisée en ce qu'**elle est constituée par la séquence nucléotidique SEQ ID n°9.

2. Oligonucléotide dont la séquence nucléotidique consiste en une séquence du gène rpoB de la bactérie *Tropheryma whippelii* d'au moins 10 nucléotides, comprise dans la séquence nucléotidique SEQ ID N° 9 et non entièrement comprise dans la séquence SEQ ID N° 3, ou une séquence complémentaire de ladite séquence.

3. Oligonucléotide dont la séquence est une séquence spécifique de la bactérie *Tropheryma whippelii,* comprenant une séquence du gène rpoB de la bactérie *Tropheryma whippelii* d'au moins 10 nucléotides, comprise dans la séquence nucléotidique SEQ ID N° 9 et non entièrement comprise dans la séquence SEQ ID N° 3, ou une séquence complémentaire de ladite séquence.

4. Oligonucléotide selon la revendication 2 ou 3, **caractérisé en ce qu'**il présente une séquence nucléotidique d'au moins 10 motifs nucléotidiques consécutifs, incluse dans l'une des séquences SEQ ID N° 10, SEQ ID N° 11 et SEQ ID N° 12, et leurs séquences complémentaires.

5. Oligonucléotide selon la revendication 2 ou 3, **caractérisé en ce qu'**il présente une séquence choisie parmi :
- les séquences d'au moins 10 nucléotides consécutifs incluses dans une séquence choisie par les séquences SEQ ID N° 5 à 8 et SEQ ID N° 17, et leurs séquences complémentaires.

6. Oligonucléotide selon la revendication 4 ou 5, **caractérisé en ce qu'**il présente une séquence choisie parmi les séquences complètes SEQ ID N° 5 à 8 , et SEQ ID N°11, 12 et 17 et leurs séquences complémentaires.

7. Sonde pour la détection, dans un échantillon biologique, de bactéries de l'espèce *Trophyrema whippelii* **caractérisée en ce qu'**elle comprend un oligonucléotide selon l'une des revendications 2 à 6.

8. Sonde selon la revendication 7, **caractérisée en ce qu'**elle est constituée par un oligonucléotide choisi parmi :
- les oligonucléotides ayant une séquence nucléotidique d'au moins 10 nucléotides consécutifs incluses dans l'une des séquences SEQ ID N° 10, SEQ ID N° 11 et SEQ ID N° 12, et leurs séquences complémentaires, et
- les oligonucléotides ayant une séquence nucléotidique choisie parmi les séquences complètes SEQ ID N° 11 et SEQ ID N° 12 et leurs séquences complémentaires.

9. Procédé de détermination de la présence ou de l'absence d'une bactérie de l'espèce *Tropheryma whippelii* dans un échantillon contenant ou susceptible de contenir des acides nucléiques d'au moins une telle bactérie, **caractérisé en ce qu'**on met en contact ledit échantillon avec au moins une sonde selon l'une des revendications 7 ou 8, puis on détermine la formation ou l'absence de formation d'un complexe d'hybridation entre ladite sonde et l'acide nucléique de l'échantillon.

10. Amorce nucléotidique utilisable pour la synthèse d'une séquence spécifique du gène *rpoB* de *Tropheryma whippelii* en présence d'une polymérase, **caractérisée en ce qu'**elle comprend un oligonucléotide selon l'une des revendications 2 à 6.

11. Amorce selon la revendication 10, **caractérisée en ce qu'**elle est constituée par un oligonucléotide choisi parmi :
- les oligonucléotides ayant une séquence nucléotidique d'au moins 10 nucléotides consécutifs incluses dans l'une des séquences SEQ ID N° 10, SEQ ID N° 11 et SEQ ID N° 12, et leurs séquences complémentaires, et
- les oligonucléotides ayant une séquence nucléotidique choisie parmi les séquences complètes SEQ ID N° 11 et SEQ ID N° 12 et leurs séquences complémentaires.

12. Procédé de détermination de la présence ou de l'absence d'une bactérie de l'espèce *Tropheryma whippelii* dans un échantillon contenant, ou susceptible de contenir des acides nucléiques d'au moins une telle bactérie, **caractérisé en ce qu'**on réalise une amplification de l'ADN de l'échantillon à l'aide de deux amorces selon l'une des revendications 10 ou 11 et on compare la séquence obtenue avec la séquence SEQ ID N° 9.

13. Procédé selon la revendication 12, **caractérisé en ce qu'**on compare la séquence du produit d'amplification obtenu en réalisant au préalable son séquençage.

14. Procédé la revendication 12 ou 13, **caractérisé en ce qu'**on réalise une amplification de l'ADN de l'échantillon à l'aide des deux amorces SEQ ID N° 11 et 12 et on compare la séquence obtenue avec la séquence SEQ ID N° 10.

## Claims

1. Total sequence of the rpoB gene of the Tropheryma whippelii bacterium, **characterised in that** it is constituted by the nucleotide sequence SEQ ID N°9.

2. Oligonucleotide whose nucleotide sequence consists of a sequence of the rpoB gene of the Tropheryma whippelii bacterium having at least 10 nucleotides, included in the nucleotide sequence SEQ ID N° 9 and not fully included in the sequence SEQ ID N° 3, or a complementary sequence of said sequence.

3. Oligonucleotide whose sequence is a specific sequence of the Tropheryma whippelii bacterium, comprising a sequence of the rpoB gene of the Tropheryma whippelii bacterium of at least 10 nucleotides, included in the sequence SEQ ID n°9 and not fully included in the sequence SEQ ID n°3, or a complementary sequence of said sequence.

4. Oligonucleotide according to claim 2 or 3, **characterised in that** it has a nucleotide sequence of at least 10 consecutive nucleotide units included in one of the sequences SEQ ID N° 10, SEQ ID N° 11 and SEQ ID N° 12, and their complementary sequences.

5. Oligonucleotide according to claim 2 or 3, **characterised in that** it has a sequence selected from among:
- the sequences of at least 10 consecutive nucleotides included in a sequence selected by the sequences SEQ ID N° 5 to 8 and SEQ ID N° 17, and their complementary sequences.

6. Oligonucleotide according to claim 4 or 5, **characterised in that** it has a sequence selected from among complete sequences SEQ ID N° 5 to 8 and SEQ ID N° 11, 12 and 17 and their complementary sequences.

7. A probe for the detection, in a biological sample, of bacteria of the species Tropheryma whippelii, **characterised in that** it comprises an oligonucleotide according to one of claims 2 to 6.

8. The probe according to claim 7, **characterised in that** it is constituted by an oligonucleotide selected from:
- the oligonucleotides having a nucleotide sequence of at least 10 consecutive nucleotides included in one of the sequences SEQ ID N° 10, SEQ ID N° 11 and SEQ ID N° 12, and their complementary sequences, and
- the oligonucleotides having a nucleotide sequence selected from among the complete sequences SEQ ID N° 11 and SEQ ID N° 12 and their complementary sequences.

9. A process for determining the presence or the absence of a bacterium of the species Tropheryma whippelii in a sample containing or capable of containing nucleic acids of at least one such bacterium, **characterised in that** said sample is put in contact with at least one probe according to one of claims 7 or 8, then formation or absence of formation of a hybridization complex between said probe and the nucleic acid of the sample is determined.

10. A nucleotide primer which can be used for synthesis of a specific sequence of the rpoB gene of Tropheryma whippelii in the presence of a polymerase, **characterised in that** it comprises an oligonucleotide according to one of claims 2 to 6.

11. The primer according to claim 10, **characterised in that** it is constituted by an oligonucleotide selected from among:
- the oligonucleotides having a nucleotide sequence of at least 10 consecutive nucleotides included in one of the sequences SEQ ID N° 10, SEQ ID N° 11 and SEQ ID N° 12, and their complementary sequences, and
- the oligonucleotides having a nucleotide sequence selected from among the complete sequences SEQ ID N° 11 and SEQ ID N° 12 and their complementary sequences.

12. The process for determining the presence or absence of a bacterium of the species Tropheryma whippelii in a sample containing, or capable of containing nucleic acids of at least one such bacterium, **characterised in that** amplification of the DNA of the sample is carried out by means of two primers according to one of claims 10 or 11 and the sequence obtained is compared with the sequence SEQ ID N° 9.

13. The process according to claim 12, **characterised in that** the sequence of the amplification product obtained is compared by running its sequencing in advance.

14. The process according to claim 12 or 13, **characterised in that** amplification of the DNA of the sample is carried out by means of the two primers SEQ ID N° 11 and 12 and the sequence obtained is compared with the sequence SEQ ID N° 10.

## Patentansprüche

1. Gesamtsequenz des Gens rpoB der Bakterie *Tropheryma whippelii,* **dadurch gekennzeichnet, daß** sie durch die Nukleotidsequenz SEQ ID Nr. 9 gebildet ist.

2. Oligonukleotid, dessen Nukleotidsequenz aus einer Sequenz des Gens rpoB der Bakterie *Tropheryma whippelii* von wenigstens zehn Nukleotiden besteht, die in der Nukleotidsequenz SEQ ID Nr. 9 enthalten ist und nicht ganz in der Sequenz SEQ ID Nr. 3 enthalten ist, oder einer komplementären Sequenz der Sequenz.

3. Oligonukleotid, dessen Sequenz eine spezifische Sequenz der Bakterie *Tropheryma whippelii* ist, mit einer Sequenz des Gens rpoB der Bakterie *Tropheryma whippelii* von wenigstens zehn Nukleotiden, die in der Nukleotidsequenz SEQ ID Nr. 9 enthalten ist und nicht ganz in der Sequenz SEQ ID Nr. 3 enthalten ist, oder einer komplementären Sequenz der Sequenz.

4. Oligonukleotid nach Anspruch 2 oder 3, **dadurch gekennzeichnet, daß** es eine Nukleotidsequenz von wenigstens zehn aufeinanderfolgenden Nukleotideinheiten, die in einer der Sequenzen SEQ IDNr. 10, SEQ ID Nr. 11 und SEQ ID Nr. 12 eingeschlossen ist, und ihre komplementären Sequenzen aufweist.

5. Oligonukleotid nach Anspruch 2 oder 3, **dadurch gekennzeichnet, daß** es eine Sequenz aufweist, die gewählt ist aus:
- den Sequenzen von wenigstens zehn aufeinanderfolgenden Nukleotiden, die in einer Sequenz eingeschlossen sind, die aus den Sequenzen SEQ ID Nr. 5 bis 8 und SEQ ID Nr. 11, 12 und 17 gewählt sind, und ihren komplementären Sequenzen.

6. Oligonukleotid nach Anspruch 4 oder 5, **dadurch gekennzeichnet, daß** es eine Sequenz aufweist, die aus den kompletten Sequenzen SEQ ID Nr. 5 bis 8 und SEQ ID Nr. 11, 12 und 17 und ihren komplementären Sequenzen gewählt ist.

7. Sonde zur Erfassung von Bakterien der Gattung *Tropheryma whippelii* in einer biologischen Probe, **dadurch gekennzeichnet, daß** sie ein Oligonukleotid nach einem der Ansprüche 2 bis 6 aufweist.

8. Sonde nach Anspruch 7, **dadurch gekennzeichnet, daß** sie durch ein Oligonukleotid gebildet ist, das gewählt ist aus:
- den Oligonukleotiden mit einer Nukleotidsequenz von wenigstens zehn aufeinanderfolgenden Nukleotiden, die in einer der Sequenzen SEQ ID Nr. 10, SEQ ID Nr. 11 und SEQ ID Nr. 12 eingeschlossen ist, und ihren komplementären Sequenzen, und
- den Oligonukleotiden mit einer Nukleotidsequenz gewählt aus den kompletten Sequenzen SEQ ID Nr. 11 und SEQ ID Nr. 12 und ihren komplementären Sequenzen.

9. Verfahren zur Bestimmung der Anwesenheit oder der Abwesenheit einer Bakterie der Gattung *Tropheryma whippelii* in einer Probe, die Nukleinsäuren wenigstens einer solchen Bakterie enthält oder enthalten kann, **dadurch gekennzeichnet, daß** man die Probe mit wenigstens einer Sonde nach einem der Ansprüche 7 oder 8 in Kontakt bringt, dann die Bildung oder die Abwesenheit der Bildung eines Hybridisierungskomplexes zwischen der Sonde und der Nukleinsäure der Probe bestimmt.

10. Nukleotid-Primer, verwendbar für die Synthese einer spezifischen Sequenz des Gens rpoB von *Tropheryma whippelii* in Anwesenheit einer Polymerase, **dadurch gekennzeichnet, daß** er ein Oligonukleotid nach einem der Ansprüche 2 bis 6 aufweist.

11. Primer nach Anspruch 10, **dadurch gekennzeichnet, daß** er durch ein Oligonukleotid gebildet ist, das gewählt ist aus:
- den Oligonukleotiden mit einer Nukleotidsequenz von wenigstens zehn aufeinanderfolgenden Nukleotiden, die in einer der Sequenzen SEQ ID Nr. 10, SEQ ID Nr. 11 und SEQ ID Nr. 12 eingeschlossen ist, und ihren komplementären Sequenzen, und
- den Oligonukleotiden mit einer Nukleotidsequenz gewählt aus den kompletten Sequenzen SEQ ID Nr. 11 und SEQ ID Nr. 12 und ihren komplementären Sequenzen.

12. Verfahren zur Bestimmung der Anwesenheit oder der Abwesenheit einer Bakterie der Gattung *Tropheryma whippelii* in einer Probe, die Nukleinsäuren wenigstens einer solchen Bakterie enthält oder enthalten kann, **dadurch gekennzeichnet, daß** man eine Amplifizierung der DNA der Probe mit Hilfe von zwei Primern nach einem der Ansprüche 10 oder 11 durchführt und die erhaltene Sequenz mit der Sequenz SEQ ID Nr. 9 vergleicht.

13. Verfahren nach Anspruch 12, **dadurch gekennzeichnet, daß** man die Sequenz des erhaltenen Amplifizierungsprodukts vergleicht, indem vorher seine Sequenzierung durchgeführt wird.

14. Verfahren nach Anspruch 12 oder 13, **dadurch gekennzeichnet, daß** man eine Amplifizierung der DNA der Probe mit Hilfe von zwei Primern SEQ ID Nr. 11 und 12 durchführt und die erhaltene Sequenz mit der Sequenz SEQ ID Nr. 10 vergleicht
